# EUROPEAN PATENT SPECIFICATION

(11) **EP 3 694 999 B1**
(45) Date of publication and mention of the grant of the patent: **01.01.2025**
(21) Application number: 18785621.6
(22) Date of filing: 12.10.2018
(51) Int. Cl.: C12P 13/00, C12P 13/04

(54) **METHODS FOR INCREASING THE TOLERANCE OF MICROBIAL CELLS TOWARDS ANTHRANILIC ACID BY LIMITING THE AMOUNT OF AMMONIA IN THE CULTURE MEDIUM**
VERFAHREN ZUR ERHÖHUNG DER TOLERANZ VON MIKROBISCHEN ZELLEN GEGENÜBER ANTHRANILSÄURE DURCH BESCHRÄNKUNG DER MENGE VON AMMONIAK IN DEM KULTURMEDIUM
PROCÉDÉS POUR AUGMENTER LA TOLÉRANCE DES CELLULES MICROBIENNES ENVERS L'ACIDE ANTHRANILIQUE EN LIMITANT LA QUANTITÉ D'AMMONIAC DANS LE MILIEU DE CULTURE

(30) Priority: 12.10.2017 EP 17196153
(43) Date of publication of application: 19.08.2020
(73) Proprietor: Covestro Deutschland AG, 51373 Leverkusen (DE)
(72) Inventor: JÄGER, Gernot, 50733 Köln (DE); KLOECKNER, Wolf, 51065 Köln (DE); BEHNKEN, Swantje, Sacramento CA 95864 (US); KLAFFL, Simon, 40597 Düsseldorf (DE); SASSI, Jamaleddine, 50739 Köln (DE)
(74) Representative: Levpat
(86) International application number: PCT/EP2018/077878
(87) International publication number: WO 2019/073035

(56) References cited:
- WO-A1-2015/124686
- WO-A1-2015/124687
- WO-A1-2017/085170
- WO-A1-2017/102853

## Description

The present invention relates to methods for increasing the concentration of anthranilic acid tolerated by a given microbial cell by limiting the concentration of ammonia in the culture medium.

Currently, there is no renewable or biologically derived source of o-aminobenzoate or the corresponding acid commercially available. Current production methods of aniline rely on chemical synthesis from petroleum-derived raw-materials. Such petroleum-derived raw materials are not renewable as opposed to raw materials which are renewable, such as the renewable resource "biomass". The chemical synthesis of aniline is a multi-step process. The several reaction steps involved in the production of aniline result in high production costs. Moreover, the conventional, i.e. chemical, synthesis of aniline is associated with hazardous intermediates, solvents, and waste products which can have substantial impacts on the environment. Non-specific side-reactions on the aromatic-ring result in the reduction of the product yield, thus further increasing the production costs. Petroleum-derived raw materials are influenced by cost fluctuations resulting from the global petroleum price.

o-aminobenzoate is a natural intermediate of the shikimate acid pathway and a precursor for the biosynthesis of the aromatic amino acid L-tryptophane. WO 2015/124687 discloses a concept of producing biologically-derived aniline in two process steps: (1) the fermentative production of o-aminobenzoate using recombinant bacteria and (2) the subsequent catalytic conversion of o-aminobenzoic acid into aniline. The recombinant bacteria used in said process belong to the family of *Corynebacterium* or *Pseudomonas.* Both bacteria produce o-aminobenzoate at pH values between 7 and 8.

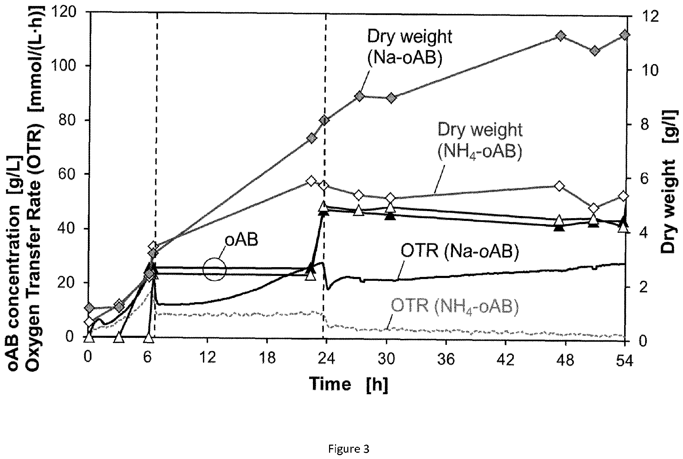

The following problem exists when producing o-aminobenzoate at pH in the range between 7 and 8: Due to the fermentative production of o-aminobenzoate which is an acid, a base such as NH₄OH, needs to be added in order to ensure a stable neutral pH. Thereby, a salt of e.g. NH₄⁺/o-aminobenzoate⁻ is produced. However, such o-aminobenzoate salts are toxic to microbial cells. According to Figure 3, the metabolic activity of bacterial cells (see OTR) is limited when NH4⁺/o-aminobenzoate concentrations of more than 25 g/L o-aminobenzoate (not including the mass of the cation)are reached and cell growth (see dry weight) stops at higher concentration (>50 g/L). This toxicity is not known for products such as glutamate or lysine.

This type of toxicity problem is typically solved by direct evolution of the applied microbial cells. First, microbial cells are exposed to increasing concentration of the toxic component (e.g. o-aminobenzoate) in repeated batch experiments or continuous fermentation trials. Thereby, the microbial cells evolve by random mutagenesis (which can be accelerated by adding mutagens) and the more resistant microbial cells survive. Secondly, the most resistant cells are isolated/selected and can be used for production.

However, many of the mechanisms underlying resistance in such microbial cells consume energy (Aindrila Mukhopadhyay. Trends in Microbiology, August 2015, Vol. 23, No. 8; Rau et al. Microb Cell Fact (2016) 15: 176; Warnecke T, Gill RT. Microbial Cell Factories. 2005; 4: 25). Thus, a certain proportion of the fermentable substrate is consumed for maintenance metabolism leading to decreased yields of o-aminobenzoate. For this reason, the high titers of o-aminobenzoate which are required for a reasonable space-time yield of the process concomitantly decrease product yield.

Although biotechnological production of o-aminobenzoate from renewable sources as a precursor for aniline production offers potential benefits, the above-described factor of toxicity diminishes the potential benefits of this process. Therefore, there is a need for alternative methods for increasing the resistance of microbial cells towards o-aminobenzoic acid.

This problem is solved by the embodiments defined in the claims and the description below.

In a first embodiment, the present invention relates to a method for cultivating at least one microbial cell capable of converting a fermentable substrate into oAB in the presence of the fermentable substrate while maintaining its metabolic activity, wherein the culture medium is characterized by a concentration of ammonia which does not exceed 200 mM, a pH between 6.0 and 8.0 and a concentration of ortho-aminobenzoic acid (oAB) of at least 40 g/l to 80 g/l.

Preferably, the concentration of oAB in this embodiment is 40 g/l to 70 g/l and more preferably 40 g/l to 60 g/l.

In this embodiment, the metabolic activity is preferably oxygen consumption as determined by the oxygen transfer rate (OTR) of the culture. The "culture" is the suspension consistting of the at least one microbial cell and the culture medium. A maintained metabolic activity of the cell or cells is indicated by an OTR of the culture which does not decrease. Preferably, a maintained metabolic activity is an increasing OTR of the culture.

Under the culture conditions set forth above, said microbial cells converts at least a proportion of the fermentable substrate into oAB. Therefore, disclosed is a method for producing ortho-aminobenzoic acid (oAB) by cultivating a microbial cell capable of converting a fermentable substrate into oAB in the presence of the fermentable substrate and under conditions suitable for the conversion of the fermentable substrate into oAB, wherein the concentration of ammonia in the culture medium does not exceed 200 mM and the concentration of oAB is at least 40 g/l.

Preferably, the concentration of oAB in this embodiment is 40 g/l to 80 g/, more preferably 40 g/l to 70 g/l and more preferably 40 g/l to 60 g/l.

The microbial cell is, preferably, a cell which is capable of biologically converting a fermentable substrate into oAB. The term "biologically converting" refers to the biochemical processes which transform one or more molecules of the fermentable substrate into one or more molecules oAB. These processes are predominantly mediated by enzymes expressed by the bacterial cell.

The term "o-aminobenzoic acid" (or oAB) as referred to in the present application relate to 2-aminobenzoic acid. This compound is also known as anthranilic acid. The person skilled in the art knows that an acid may be present in its protonated form as neutral substance or deprotonated as anion. In aqueous solution a part of the acid is protonated and a part is present as anion. The ratio between protonated acid and anion depends on the pH of the solution and the dissociation constant Kₐ of the acid in question. Unless indicated otherwise, the term "o-aminobenzoic acid" as used in this application always refers to both the protonated acid as well as the corresponding anion.

The microbial cell used in the present invention may be a naturally occurring strain, i.e. a microbial strain which is without any further human interaction, particularly without genetic manipulation, capable of converting a fermentable substrate into oAB. However, in a preferred embodiment of the present invention, it is a microbial cell which gained the aforementioned capability in the process of genetic manipulation or a microbial cell, where such methods were used to improve a pre-existing capability.

The term "genetic modification" within the meaning of the invention refers to changes in nucleic acid sequence of a given gene of a microbial host as compared to the wild-type sequence. Such a genetic modification can comprise deletions as well as insertions of one or more deoxyribo nucleic acids. Such a genetic modification can comprise partial or complete deletions as well as insertions introduced by transformations into the genome of a microbial host. Such a genetic modification can produce a recombinant microbial host, wherein said genetic modification can comprise changes of at least one, two, three, four or more single nucleotides as compared to the wild type sequence of the respective microbial host. For example, a genetic modification can be a deletion or insertion of at least one, two, three, four or more single nucleotides or a transformation of at least one, two, three, four or more single nucleotides. A genetic modification can have the effect of e.g. a reduced expression of the respective gene or of e.g. an enhanced expression of the respective gene.

The microbial cell is a prokaryotic cell or an eukaryotic cell. Preferably, the prokaryotic cell is a bacterial cell. Preferred bacterial cells belong to genera *Corynebacterium, Mycobacterium, Bacillus, Pseudomonas, Escherichia, and Vibrio.* More preferred are *Corynebacterium glutamicum* and *Pseudomonas putida.* Most preferred is *Corynebacterium glutamicum* ATCC 13032. Preferred eukaryotic cells belong to the order *Saccharomycetales or the genus Aspergillus.* More preferably, they belong to the species *Ashbya gossypii, Pichia pastoris, Hansenula polymorpha, Yarrowia lipolytica, Zygosaccharomyces bailii, Kluyveromyces marxianus* and *Saccharomyces cerevisiae.* Most preferably, the yeast is *Saccharomyces cerevisiae.*

It is particularly preferred that said microbial cell is characterized by a genetic modification of the *trpD* gene which prevents or decreases the expression of said gene and/or which leads to a gene product with decreased or without enzymatic activity. The person of average skill in the art can easily generate such microbial cells using conventional genetic methods.

Recombinant bacterial cells which are particularly suitable for the method of the present invention are disclosed in WO 2015/124687.

The term "cultivating" refers to the incubation of the microbial cell under conditions which facilitate metabolic activity. Such conditions are known to the person skilled in the art. Said conditions minimally encompass presence of the microbial cell in a medium suitable for growth of the cell at temperatures which allow cell proliferation, presence of a fermentable substrate and presence of oxygen. Preferably, said metabolic activity is oxygen consumption. More preferably, the metabolic activity is measured as one or more parameter selected from the group consisting of oxygen consumption, glucose consumption, oAB-production and growth rate. Most preferably, the metabolic activity is the biological conversion of the fermentable substrate to oAB or the growth rate.

Preferably, the cultivation is performed in a culture medium having a pH between 6.0 and 8.0.

A fermentable substrate as understood by the present application is any organic compound or mixture of organic compounds which can be utilized by the microbial cell to produce o-aminobenzoic acid in the presence or absence of oxygen. Preferred fermentable substrates additionally serve as energy and carbon sources for the growth of the microbial cell. Preferred fermentable substrates are processed sugar beet, sugar cane, starch-containing plants and lignocellulose. Also preferred as fermentable substrate are glycerol and C1-compounds, preferably CO, and fermentable sugars. A preferred fermentable sugar is glucose.

The term "culture medium" is generally understood in the art. It refers to an aqueous solution which provides conditions which allow metabolic activity of the microbial cell. Said conditions are physical or physicochemical such as temperature, concentration of dissolved oxygen, ion strength and pH. They are also chemical and include the concentration of the different nutrients required by the microbial cell for its activity. The person skilled in the art can adapt these conditions to the needs of a particular microbial cell based on the common knowledge available for the particular microbial cell.

The term "ammonia" refers to ammonia in its neutral form, i.e. NH₃, as well as to ammonium, i.e. NH₄⁺. "Ammonia concentrations", hence, always refer to the sum of the concentrations of both species.

Aqueous ammonia is a popular buffer in biotechnological applications. In the production of organic acids such as oAB it can be used to maintain a stable pH despite increasing concentrations of the acidic fermentation product. However, in the study underlying the present invention it was surprisingly found that the absence of high ammonia concentrations due to the use of sodium hydroxide as buffer substance lead to a marked increase of the oAB-concentration tolerated by the microorganism in question.

Therefore, the concentration of ammonia during the production of oAB by a microbial cell must be limited in order to concomitantly limit product toxicity of oAB. The concentration of ammonia present during the production of oAB does not exceed 200 mM. More preferably it reaches a maximum of 100 mM and most preferably of 50 mM. It is to be understood that the addition of aqueous ammonia or ammonium salts from stock solutions as nitrogen source or as component of a buffer system may lead to concentrations which exceed the aforementioned values locally and for a limited period of time because the even distribution of the added liquid in a fermentation vessel requires a certain amount of time. The same applies for the addition of ammonium salts in solid form, where the dissolution of the salt may lead to local peaks of ammonia concentration before the dissolved salt is diluted.

Thus, the term "locally" refers to a proportion of not more than 10 % of the total volume of the culture medium. Any peak of the ammonia concentration in the culture medium does not last for more than 15 minutes before the mixing of the culture medium leads to a distribution of the added compounds in the vessel so that the above-defined threshold levels are not exceeded.

Since smaller concentrations of ammonia do not cause excessive toxicity of oAB, the culture medium may contain 0.5 mM to 200 mM and most preferably 0.5 mM to 100 mM ammonia. Preferably, the lower limit of the concentration of ammonia is 10 mM. Thus, ammonia can still be used as a part of the buffer system and it can be used as a cheap and convenient nitrogen source.

However, in another preferred embodiment of the present invention no ammonia is actively added to the culture medium so that the concentration of ammonia does not exceed 5.0 mM, preferably 1.0 mM and more preferably 0.5 mM. In this embodiment, nitrogen sources other than ammonia must be used. Alternative nitrogen sources are well known to the person skilled in the art. Preferred alternative nitrogen sources are urea, amino acids, peptides as well as complex mixtures which comprise the aforementioned compounds. Mixtures comprising alternative nitrogen sources are, preferably, yeast extract, tryptone, casamino acids and corn steep liquor. Preferred amino acids are glutamine, threonine, alanine, asparagine and serine.

In a preferred embodiment of the present invention low concentrations of ammonia are supplemented with alternative nitrogen sources. This is done preferably if the total ammonia concentration at any point in time of the cultivation is in the range between 0.5 and 250 mM, more preferably 0.5 to 100 mM. A preferred alternative nitrogen source to be used for supplementing the aforementioned ammonia concentration is urea.

Under the above-defined culture conditions, oAB-concentrations of at least 20 g/l, preferably at least 30 g/l and most preferably at least 40 g/l are reached.

Preferably, under the above-defined culture conditions the microbial biomass reaches a biomass of at least 6 g/l, more preferably at least 9 g/l.

In a particular preferred aspect of the disclosure the microbial biomass reaches a biomass of at least 6 g/l, more preferably at least 9 g/l, under the above-defined culture conditions and in the presence of at least 30 g/l oAB, more preferably at least 40 g/l oAB.

Advantageously, the microbial cell maintains its metabolic activity up to concentrations of at least 30 g/l oAB, more preferably at least 40 g/l oAB and most preferably at least 50 g/l oAB if the concentration of ammonia in the culture medium most preferably does not exceed 100 mM. The cell maintains its metabolic activity if said metabolic activity is at least 50 % of the activity in the absence of oAB. Preferably, metabolic activity is measured as one or more parameter selected from the group consisting of oxygen consumption, glucose consumption, oAB-production and growth rate.

It is to be understood that any sudden increase of oAB concentration causes a transient decrease of the metabolic activity of the microbial cell (see examples). During the process of oAB production this effect is unlikely to be encountered because oAB concentrations increase gradually so that the microbial cell has time to adapt. However, addition of oAB for testing purposes may have this effect. Therefore it is preferred that the metabolic activity of a microbial cell is measured at least two hours after any sharp increase of oAB concentration, e.g. caused by the addition of oAB to the culture medium. Otherwise the true and lasting effect of oAB on the activity may be overestimated for the given conditions.

In one preferred embodiment of the present invention aqueous ammonia is replaced by the base of an alkali metal ion as buffering agent during the cultivation of the microbial cell. Said base of an alkali metal is preferably sodium hydroxide.

In another preferred embodiment of the present invention no buffering agent is added during the cultivation of the microbial cell. A buffering agent can be omitted if the culture medium contains sufficient concentrations of CaCO₃ from the beginning of the fermentation process or if urea is used as alternative nitrogen source. Based on the amount of CaCO₃ which is required to neutralize a given amount of oAB and the acceptable maximum drop in pH which is acceptable, the initial concentration of oAB can be calculated. If the addition of a buffering agent during the fermentation is to be omitted, 10 to 30 g/l, more preferably 15 to 25 g/l CaCO₃ are added to the culture medium before the start of the fermentation.

In another preferred embodiment the present invention relates to the use of a culture medium with an ammonia concentration not exceeding 200 mM for the biological conversion of a fermentable carbon source into oAB by a microbial cell capable of said conversion.

All definitions given above for the method of the invention also apply to this embodiment unless otherwise specified.

The ammonia concentration in the culture medium does not exceed 200 mM. Even more preferably, it does not exceed 100 mM. Most preferably, it does not exceed 5 mM.

Disclosed is the use of a culture medium with an ammonia concentration not exceeding 10 mM in order to increase the tolerance of microbial cells towards oAB.

All definitions given above for the method of the invention also apply to this embodiment unless otherwise specified.

The term "use of a culture medium in order to increase the tolerance of microbial cells towards oAB"

The following examples are only intended to illustrate the invention. They shall not limit the scope of the claims in any way.

### Examples

The figures show:

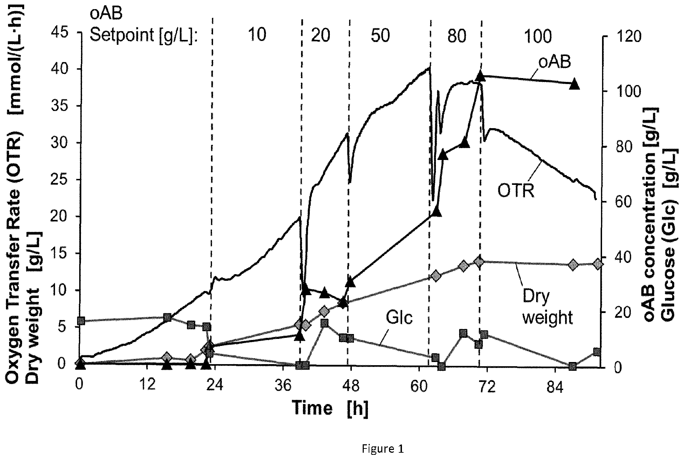

- **Figure 1:**: Evaluation of the maximum tolerable oAB concentration during batch cultivation in a 1 L lab-scale bioreactor with a modified C. *glutamicum* production strain. Addition of 20 mL of a 500 g/L oAB stock solution (oAB dissolved with NaOH at pH 7) after 23 h and 39 h, 48 h (two times), 64 h (two times) and 71 h (two times) and addition of glucose solution after 40 h, 48 h, 63 h, 65 and 71.5 h to prevent a glucose limitation. Initial cultivation volume VL=1 L, temperature 30°C, pH =7 controlled by adding NH4OH (10 w% NH3) during cultivation, gassing rate with air = 0.2 L/min, pO₂ controlled at 30% air saturation by adjusting the stirrer speed between 200 and 1200 rpm.

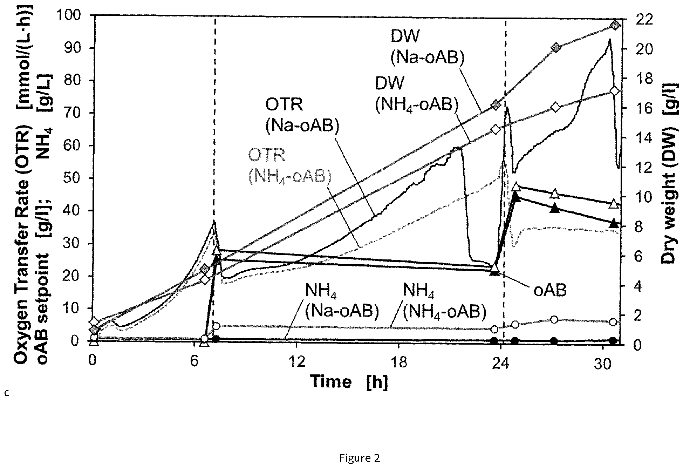

- **Figure 2:**: Influence of Na-oAB and NH₄-oAB addition on oAB resistance during batch cultivation in 1 L scale with C. *glutamicum* ATCC 13032. Filled symbols: addition of 40 mL of 500 g/L oAB stock solution (oAB dissolved with NaOH at pH 7) after 7 h and 24 h. Open symbols: addition of 40 mL of 500 g/L oAB stock solution (oAB dissolved with NH₄OH at pH 7) after 7 h and 24 h. Both bioreactors: addition of 36 g/L glucose (added as stock solution) after 7.5 h and 24.5 h to prevent a glucose limitation. Initial cultivation volume VL=1 L, temperature 30°C, pH =7 controlled with NH₄OH (10 w% NH₃), gassing rate with air = 0.2 L/min, pO₂ controlled at 30% air saturation by adjusting the stirrer speed between 200 and 1200 rpm.
- **Figure 3:**: Influence of Na-oAB and NH₄-oAB addition on oAB resistance during batch cultivation in 1 L scale with C. *glutamicum* ATCC 13032. Filled symbols: addition of 40 mL of 500 g/L oAB stock solution (oAB dissolved with NaOH at pH 7) after 6.4 h and 23.6 h. Open symbols: addition of 40 mL of 500 g/L oAB stock solution (oAB dissolved with NH₄OH at pH 7) after 6.4 h and 23.6 h. Initial cultivation volume VL=1 L, temperature 30°C, pH =7 controlled with NH₄OH (10 w% NH₃), gassing rate with air = 0.2 L/min, pO₂ controlled at 30% air saturation by adjusting the stirrer speed between 200 and 1200 rpm.

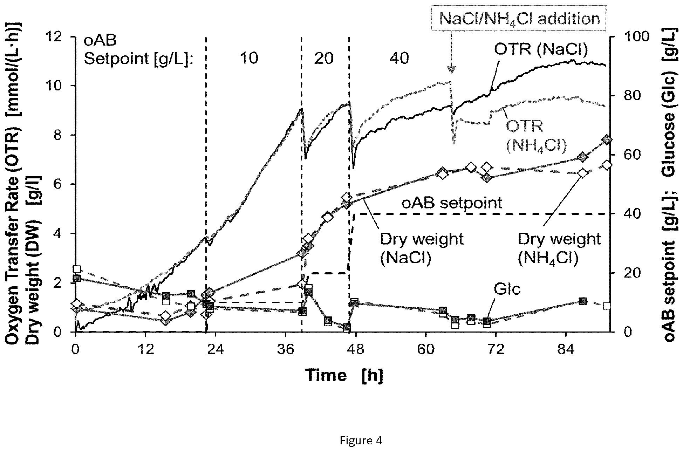

- **Figure 4:**: Influence of NaCl and NH₄Cl addition on oAB resistance during batch cultivation in 1 L scale with a modified C. *glutamicum* production strain. Addition of 20 mL of 500 g/L oAB stock solution (oAB dissolved with NaOH at pH 7) after 23 h, 39 h and 47 h (two times) and addition of glucose after 19 h, 37 h, 47 h and 71 h to prevent a glucose limitation. Solid lines: reactor 1, addition of 70 mL of a 244 g/L NaCl stock solution after 64 h. Dashed lines: reactor 2, addition of 70 mL of a 223 g/L NH₄Cl-stock solution after 64 h. Initial cultivation volume VL=1 L, temperature 30°C, pH =7 controlled with aqueous NH₄OH (10 w% NH₃), gassing rate with air = 0.2 L/min, pO₂ controlled at 30% air saturation by adjusting the stirrer speed between 200 rpm and 1200 rpm.

### Example 1: Evaluation of the maximum tolerable oAB concentration of a modified C. glutamicum production strain.

The maximum tolerable oAB concentration in the presence of sodium ions in the medium was tested with a modified C. *glutamicum production* strain. This strain was derived from *Corynebacterium glutamicum* ATCC 13032 by evolutionary engineering in order to increase its resistance against oAB. This strain was then genetically modified to make it capable of oAB-production and comprised the following modifications. The incorporated modifications have the effect of reduced expression of the *trpD* gene, encoding anthranilate phosphoribosyl transferase, knock out of gene *ppc,* encoding PEP Carboxylase, constitutive overexpression of heterologous *aroG*^{D146N} and *trpEG*^{S40F} genes from *E. coli,* encoding feedback resistant DAHP synthase and anthranilate synthase, respectively, constitutive overexpression of the gene *aroL* from *E. coli,* encoding shikimate kinase.

One bioreactor with a nominal volume of 1 L was filled with sterile cultivation medium including an initial amount of 20 g/L glucose, 5 g/L (NH₄)₂SO₄, 1 g/L KH₂PO₄, 1 g/LK₂HPO₄, 0.25 g/L MgSO₄·7 H₂O, 0.01 g/L CaCl₂·2 H₂O, 2 mg/L biotin (vitamin B7), 0.03 g/L protocatechuic acid (3,4-Dihydroxybenzoic acid), 0.01 g/L MnSO₄·H₂O. 0.01 g/L FeSO₄·7H₂O, 1 mg/L ZnSO₄·7H₂O, 0.2 mg/L CuSO₄·5H₂O and 0.02 mg/L NiCl₂·6H₂O.

The preculture medium for the cultivation in shake flasks contained additionally 42 g/L MOPS buffer, 3.7 g/L brain heart infusion broth, 5 g/L urea (CH₄N₂O) and 20 g/L (NH₄)₂SO₄ (instead of 5 g/L). The preculture was cultivated in 300 mL shake flasks with a liquid volume of 25 mL at a temperatur of 28 °C and a shaking frequency of 180 rpm until OD₆₀₀ > 20 was reached.

The cultivation was performed in a lab scale bioreactor with an initial cultivation volume of 1 L. Temperature was controlled at 30°C and pH was kept constant at pH=7 by adding aqueous NH₄OH solution (10 w% NH₃) during the fermentation. The gassing rate was adjusted to 0.2 L/min air and the dissolved oxygen tension was controlled at 30% air saturation by controlling the stirrer speed between 200 rpm and 1200 rpm. Results of the cultivation are shown in Figure 1. The oAB concentration was increased stepwise by adding 20 mL of a 500 g/L oAB stock solution (oAB dissolved with NaOH at pH 7) after 23 h, 39 h, 48 h (two times), 64 h (two times) and 71 h (two times). Glucose was added as stock solution after 40 h, 48 h, 63 h, 65 and 71.5 h to prevent a glucose limitation. An increase in biomass concentration was observed even at an oAB concentration of 80 g/L as shown in Figure 1. Increasing the oAB concentration from 80 g/L to 100 g/L after 71 h resulted in a decrease of the metabolic activity (indicated by the declining OTR signal) and no further increase in biomass was observed at that point. With this experiment it was demonstrated that growth of the C. *glutamicum* production strain in the presence of 80 g/L oAB can be achieved by adding oAB as sodium salt instead of ammonium salt to the bioreactor and, in this way, avoiding high ammonium concentrations

### Example 2: Comparison of the metabolic activity after NH4-oAB and Na-oAB addition during the cultivation of C. glutamicum ATCC 13032

A *C. glutamicum* strain derived from ATCC 13032 was used to compare the metabolic activity after NH4-oAB and Na-oAB addition during the cultivation. This strain was derived from *Corynebacterium glutamicum* ATCC 13032 by evolutionary engineering in order to increase its resistance against oAB. It was not further genetically modified. Thus it was not capable of oAB-production. For this purpose, two 1 L bioreactors were filled with sterile cultivation medium including the following initial concentrations: 20 g/L glucose, 5 g/L (NH₄)₂SO₄, 1 g/L KH₂PO₄, 1 g/L K₂HPO₄,0.25 g/L MgSO₄·7 H₂O, 0.01 g/L CaCl₂·2 H₂O, 2 mg/L biotin (vitamin B7), 0.03 g/L protocatechuic acid (3,4-Dihydroxybenzoic acid), 0.01 g/L MnSO₄·H₂O, 0.01 g/L FeSO₄·7H₂O, 1 mg/L ZnSO₄·7H₂O, 0.2 mg/L CuSO₄·5H₂O and 0.02 mg/L NiCl₂·6H₂O.

The preculture medium for the cultivation in shake flasks contained additionally 42 g/L MOPS buffer, 3.7 g/L brain heart infusion broth, 5 g/L urea (CH₄N₂O) and 20 g/L (NH₄)₂SO₄ (instead of 5 g/L). The preculture was cultivated in 300 mL shake flasks with a liquid volume of 25 mL at a temperatur of 28 °C and a shaking frequency of 180 rpm until OD₆₀₀ > 20 was reached.

Results for dry biomass, oAB and NH₄ concentrations and the Oxygen Transfer Rate (OTR) signals are shown in Figure 2. After addition of 40 mL of a 500 g/L oAB stock solution (oAB dissolved with NaOH at pH 7) at a cultivation time of 7 h and 24 h to reactor 1, the biomass concentration and OTR signal continued to increase (filled symbols in Figure 2). In contrast to that, the addition of NH₄-oAB (added by injection of 40 mL of a 500 g/L oAB stock solution containing oAB dissolved with NH₄OH at pH 7) to reactor 2 after 24 h resulted in a constant OTR signal and a reduced biomass accumulation (open symbols in Figure 2). This experiment shows that the replacement of ammonia by sodium as counter ion for oAB increases the tolerance of C. *glutamicum* ATCC 13032 towards oAB.

### Example 3: Comparison of the metabolic activity after NH₄-oAB and Na-oAB addition during the cultivation of C. glutamicum ATCC 13032

The influence of ammonium was tested with *C. glutamicum* ATCC 13032 (without further genetic modifications, not modified by evolutionary engineering) using two 1 L bioreactors filled with sterile cultivation medium including the following initial concentrations: 20 g/L glucose, 5 g/L (NH₄)₂SO₄, 1 g/L KH₂PO₄, 1 g/L K₂HPO₄, 0.25 g/L MgSO₄·7 H₂O, 0.01 g/L CaCl₂·2 H₂O, 2 mg/L biotin (vitamin B7), 0.03 g/L protocatechuic acid (3,4-Dihydroxybenzoic acid), 0.01 g/L MnSO₄·H₂O, 0.01 g/L FeSO₄·7H₂O, 1 mg/L ZnSO₄·7H₂O, 0.2 mg/L CuSO₄·5H₂O and 0.02 mg/L NiCl₂·6H₂O.

The preculture medium for the cultivation in shake flasks contained additionally 42 g/L MOPS buffer, 3.7 g/L brain heart infusion broth, 5 g/L urea (CH₄N₂O) and 20 g/L (NH₄)₂SO₄ (instead of 5 g/L). The preculture was cultivated in 300 mL shake flasks with a liquid volume of 25 mL at a temperatur of 28 °C and a shaking frequency of 180 rpm until OD₆₀₀ > 20 was reached.

During the fermentation the temperature was controlled at 30°C and pH was kept constant at pH=7 by adding aqueous NH₄OH solution (10 w% NH₃). The gassing rate was adjusted to 0.2 L/min air and the dissolved oxygen tension was controlled at 30% air saturation by adjusting the stirrer speed between 200 rpm and 1200 rpm.

Results for dry biomass and oAB concentrations and the related signals for the Oxygen Transfer Rate (OTR) are shown in Figure 3. 40 mL of a 500 g/L oAB stock solution (oAB dissolved with NaOH at pH 7) was added to reactor 1 after 6.4 h and 23.6 h (filled symbols) and 40 mL of a 500 g/L oAB stock solution (oAB dissolved with NH₄OH at pH 7) was added after 6.4 h and 23.6 h to reactor 2 (open symbols)

As shown in Figure 3, the biomass accumulation continued after Na-oAB was added to reactor 1 (filled symbols in Figure 3). In contrast to that, the addition of NH₄-oAB to reactor 2 caused a growth inhibition after 24 h (open symbols in Figure 3). From this it follows that the toxicity of oAB on *C. glutamicum* ATCC 13032 in presence of high sodium concentrations is reduced as compared to the toxicity in presence of high ammonium concentrations.

### Example 4: Comparison of the metabolic activity after NaCl and NH₄Cl addition during cultivation of a modified C. glutamicum production strain

The strain was modified as explained in example 1. To evaluate whether an increased tolerance towards oAB is caused by the absence of high ammonium concentrations or by the presence of sodium in the medium, the influence of NaCl and NH₄Cl addition on the toxicity of oAB was tested. Two bioreactors with a nominal volume of 1 L were filled with sterile cultivation medium including the following initial concentrations: 20 g/L glucose, 5 g/L (NH₄)₂SO₄, 1 g/L KH₂PO₄, 1 g/L K₂HPO₄, 0.25 g/L MgSO₄·7 H₂O, 0.01 g/L CaCl₂·2 H₂O, 2 mg/L biotin (vitamin B7), 0.03 g/L protocatechuic acid (3,4-Dihydroxybenzoic acid), 0.01 g/L MnSO₄·H₂O. 0.01 g/L FeSO₄·7H₂O, 1 mg/L ZnSO₄·7H₂O, 0.2 mg/L C_{U}SO₄·5H₂O and 0.02 mg/L NiCl₂·6H₂O.

The preculture medium for the cultivation in shake flasks contained additionally 42 g/L MOPS buffer, 3.7 g/L brain heart infusion broth, 5 g/L urea (CH₄N₂O) and 20 g/L (NH₄)₂SO₄ (instead of 5 g/L). The preculture was cultivated in 300 mL shake flasks with a liquid volume of 25 mL at a temperatur of 28 °C and a shaking frequency of 180 rpm until OD₆₀₀ > 20 was reached.

The cultivation was performed in lab scale bioreactors with an initial cultivation volume of 1 L. Temperature was controlled at 30°C and the pH value was kept constant at pH 7 by adding aqueous NH₄OH solution (10 w% NH₃) during the fermentation. The gassing rate was adjusted to 0.2 L/min air and the dissolved oxygen tension was controlled at 30% air saturation by adjusting the stirrer speed between 200 rpm and 1200 rpm. Two bioreactors were inoculated with the production strain. The cultivation results are shown in Figure 4. The oAB concentration was increased stepwise in both bioreactors by adding 20 mL of a 500 g/L oAB stock solution (Na-oAB) after 23 h, 39 h and 47 h (two times). Glucose was added after 19 h, 37 h, 47 h and 71 h to a prevent glucose limitation. After a cultivation time of 64 h, a volume of 70 mL water containing 244 g/L NaCl was added to bioreactor 1 (solid lines in Figure 4) resulting in an added amount of 292 mmol Na⁺ ions. An equimolar amount of NH₄Cl was added to reactor 2 by adding 70 mL of a 223 g/L NH₄Cl stock solution after 64 h (dashed lines). The online signals for the Oxygen Transfer Rates (OTR) for both bioreactors are shown in Figure 4. The addition of NaCl had no effect on the OTR signal of reactor 1. In contrast to that, the OTR signal of reactor 2 decreased by about 20 % after NH₄Cl solution was added (Figure 4). The drop in the metabolic activity affected the final dry weight concentration that reached 7.8 g/L in reactor 1 and 6.8 g/L in reactor 2 after a cultivation time of 91 hours. The results demonstrated that increased ammonium concentrations lead to a reduced metabolic activity of the *C. glutamicum* production strain.

The effects described above can be generalized at least to the genus Corynebacterium. A strain which underwent evolutionary engineering, a method which changes the genetic material of an organism in a random fashion, shows the same behavior as the strain used in example 3. Thus, the effect seems to be based on some rather fundamental functions of the cell which are not easily altered.

## Claims

1. A method for cultivating at least one cell of *Corynebacterium glutamicum* capable of converting a fermentable substrate into oAB in the presence of the fermentable substrate while maintaining its metabolic activity, wherein the culture medium is **characterized by** a concentration of ammonia which does not exceed 200 mM, a pH between 6.0 and 8.0 and a concentration of ortho-aminobenzoic acid (oAB) of 40 g/l to 80 g/l.

2. The method of claim 1, wherein maintained metabolic activity is indicated by an oxygen transfer rate of the culture which does not decrease.

3. The method of claim 1, wherein the concentration of oAB is 40 g/l to 60 g/l and at least a proportion of the fermentable substrate is converted to oAB by the at least one cell.

4. The method of claim 2, wherein the concentration of ammonia in the culture medium does not exceed 5 mM.

5. The method of claim 3 or 4, wherein the culture medium contains a nitrogen source which is not ammonia.

6. The method of claim 5, wherein the nitrogen source is urea.

7. The method of claim 5, wherein the nitrogen source is selected from the group consisting of glutamine, alanine, asparagine, serine, threonine, peptides, and complex mixtures containing nitrogen.

8. The method of any one of claims 3 to 7 comprising a further step of adding the base of an alkali metal ion to the culture medium as buffering agent.

9. The method of anyone of claims 1 to 8, wherein microbial biomass reaches at least 6 g/l dry weight.

10. Use of a culture medium with an ammonia concentration not exceeding 200 mM, a pH between 6.0 and 8.0 and a concentration of oAB of 40 g/l to 60 g/l for the biological conversion of a fermentable carbon source into oAB by a cell of *Corynebacterium glutamicum* capable of said conversion.

## Patentansprüche

1. Verfahren zur Kultivierung wenigstens einer Zelle von *Corynebacterium glutamicum,* die zum Umwandeln eines fermentierbaren Substrats in oAB in der Lage ist, in Gegenwart des fermentierbaren Substrats unter Aufrechterhaltung ihrer Stoffwechselaktivität, wobei das Kulturmedium durch eine Ammoniakkonzentration, die 200 mM nicht überschreitet, einen pH-Wert zwischen 6,0 und 8,0 und eine Konzentration an ortho-Aminobenzoesäure (oAB) von wenigstens 40 g/l bis 80 g/l gekennzeichnet ist.

2. Verfahren nach Anspruch 1, wobei die aufrechterhaltene Stoffwechselaktivität angezeigt wird durch eine Sauerstofftransferrate der Kultur, die nicht abnimmt.

3. Verfahren nach Anspruch 1, wobei die Konzentration von oAB 40 g/l bis 60 g/l beträgt, und wenigstens ein Teil des fermentierbaren Substrats durch die wenigstens eine Zelle in oAB umgewandelt wird.

4. Verfahren nach Anspruch 2, wobei die Konzentration von Ammoniak im Kulturmedium 5 mM nicht überschreitet.

5. Verfahren nach Anspruch 3 oder 4, wobei das Kulturmedium eine Stickstoffquelle enthält, bei der es sich nicht um Ammoniak handelt.

6. Verfahren nach Anspruch 5, wobei es sich bei der Stickstoffquelle um Harnstoff handelt.

7. Verfahren nach Anspruch 5, wobei die Stickstoffquelle ausgewählt ist aus der Gruppe bestehend aus Glutamin, Alanin, Asparagin, Serin, Threonin, Peptiden und komplexen Gemischen, die Stickstoff enthalten.

8. Verfahren nach einem der Ansprüche 3 bis 7, umfassend einen weiteren Schritt des Zugebens der Base eines Alkalimetallions zu dem Kulturmedium als Puffermittel.

9. Verfahren nach einem der Ansprüche 1 bis 8, wobei die mikrobielle Biomasse wenigstens 6 g/l Trockengewicht erreicht.

10. Verwendung eines Kulturmediums mit einer Ammoniakkonzentration, die 200 mM nicht überschreitet, einem pH-Wert zwischen 6,0 und 8,0 und einer Konzentration an oAB von 40 g/l bis 60 g/l für die biologische Umwandlung einer fermentierbaren Kohlenstoffquelle in oAB durch eine Zelle von *Corynebacterium glutamicum,* die zu dieser Umwandlung in der Lage ist.

## Revendications

1. Procédé pour la culture d'au moins une cellule de *Corynebacterium glutamicum* capable de convertir un substrat pouvant être fermenté en oAB en la présence du substrat pouvant être fermenté tout en maintenant son activité métabolique, le milieu de culture étant **caractérisé par** une concentration en ammoniaque qui ne dépasse pas 200 mM, un pH compris entre 6,0 et 8,0 et une concentration d'acide ortho-aminobenzoïque (oAB) de 40 g/l à 80 g/l.

2. Procédé selon la revendication 1, une activité métabolique maintenue étant indiquée par un taux de transfert d'oxygène de la culture qui ne diminue pas.

3. Procédé selon la revendication 1, la concentration d'oAB étant de 40 g/l à 60 g/l et au moins une proportion du substrat pouvant être fermenté étant convertie en oAB par l'au moins une cellule.

4. Procédé selon la revendication 2, la concentration en ammoniaque dans le milieu de culture ne dépassant pas 5 mM.

5. Procédé selon la revendication 3 ou 4, le milieu de culture contenant une source d'azote qui n'est pas l'ammoniaque.

6. Procédé selon la revendication 5, la source d'azote étant l'urée.

7. Procédé selon la revendication 5, la source d'azote étant choisie dans le groupe constitué par la glutamine, l'alanine, l'asparagine, la sérine, la thréonine, des peptides, et des mélanges complexes contenant de l'azote.

8. Procédé selon l'une quelconque des revendications 3 à 7 comprenant une étape supplémentaire d'ajout de la base d'un ion de métal alcalin au milieu de culture comme agent tampon.

9. Procédé selon l'une quelconque des revendications 1 à 8, une biomasse microbienne atteignant au moins 6 g/l de poids sec.

10. Utilisation d'un milieu de culture comportant une concentration en ammoniaque ne dépassant pas 200 mM, un pH compris entre 6,0 et 8,0 et une concentration d'oAB de 40 g/l à 60 g/l pour la conversion biologique d'une source de carbone pouvant être fermentée en oAB par une cellule de *Corynebacterium glutamicum* capable de ladite conversion.
